# EUROPEAN PATENT APPLICATION

(11) **EP 3 858 596 A1**
(43) Date of publication of application: **04.08.2021**
(21) Application number: 19865633.2
(22) Date of filing: 27.09.2019
(51) Int. Cl.: B32B 3/30, A61L 27/04, A61L 27/30, A61L 27/54, A61L 31/02, A61L 31/08, A61L 31/16, B32B 9/00, C25D 11/08

(54) **ANTIMICROBIAL MATERIAL, LAYERED BODY, ANTIMICROBIAL LAYERED BODY, MEDICAL MEMBER, ANTIMICROBIAL MATERIAL PRODUCTION METHOD, ANTIMICROBIAL LAYERED BODY PRODUCTION METHOD, AND ANTIMICROBIAL METHOD**

(30) Priority: 28.09.2018 JP 2018183510; 07.08.2019 JP 2019145611
(71) Applicant: Mitsubishi Chemical Corporation, Chiyoda-ku Tokyo 100-8251 (JP)
(72) Inventor: MURATA, Takaaki, Tokyo 100-8251 (JP); MASUDA, Hideki, Hachioji-shi, Tokyo 192-0397 (JP); YANAGISHITA, Takashi, Hachioji-shi, Tokyo 192-0397 (JP)
(74) Representative: Hoffmann Eitle
(86) International application number: PCT/JP2019/038349
(87) International publication number: WO 2020/067500

(57) **Abstract**

An antimicrobial layered body includes: a non-metal substrate; and a metal oxide layer, in which the metal oxide layer is present on an outermost surface, the metal oxide layer contains an anion, and a total abundance ratio of at least one atom of a sulfur atom, a phosphorus atom, and a carbon atom which are derived from the anion is 1.0 atm% or more when analyzed by XPS.

## Description

### [Technical Field]

The present invention relates to an antimicrobial material, a layered body, an antimicrobial layered body, a medical member, an antimicrobial material production method, an antimicrobial layered body production method, and an antimicrobial method.

Priority is claimed on Japanese Patent Application No. 2018-183510, filed on September 28, 2018, and Japanese Patent Application No. 2019-145611, filed on August 7, 2019, the contents of which are incorporated herein by reference.

### [Background Art]

Antimicrobial substances such as antibiotics and synthetic antimicrobial agents are used for purpose of treating human and livestock diseases, improving a productivity of agricultural, livestock, and marine products, storing foods, and the like, and are used as medicines, veterinary medicines, pesticides, feed additives, food additives, and the like.

In recent years, emergence of drug-resistant microorganisms such as drug-resistant bacteria and drug-resistant viruses which have resistance to drugs such as antimicrobial substances and antiviral agents and for which the drugs do not work or become difficult to work has become a problem. Therefore, use of drugs for the purpose of suppressing growth of microorganisms tends to be restricted.

For example, the followings have been proposed as an article capable of exhibiting an antimicrobial effect without using a drug.
(1) An antimicrobial article including, on a surface, a microprotrusion structure in which a plurality of microprotrusions are arranged, an average distance between adjacent microprotrusions is 30 to 90 nm, and an average aspect ratio of the microprotrusions is 3.0 to 6.25 (Patent Document 1).
(2) An antimicrobial article including a fine uneven layer made of a cured product of a resin composition, the layer having, on a surface, a microprotrusion structure in which a plurality of microprotrusions are arranged, in which an average distance between adjacent microprotrusions is 90 to 500 nm, an average aspect ratio of the microprotrusions is 1.0 or more and less than 3.0, and a static contact angle of water on the surface of the fine uneven layer is 30° or smaller (Patent Document 2).
(3) A mold growth control member including a fine uneven layer made of a cured product of a resin composition, the layer having, on a surface, a microprotrusion structure in which a plurality of microprotrusions are arranged, in which an average distance between adjacent microprotrusions is 50 to 500 nm (Patent Document 3).
(4) An antimicrobial and antifungal article including a fine uneven layer having, on a surface, a microprotrusion structure in which a plurality of microprotrusions are arranged, in which an average distance between adjacent microprotrusions is 1 µm or smaller, a height of the microprotrusion is 80 to 1000 nm, and a ratio (Wt/Wb) of a width Wt at the height of 97% of the microprotrusion to a width Wb at the bottom is 0.5 or less (Patent Document 4).
(5) An antimicrobial article including a fine uneven layer having, on a surface, a microprotrusion structure in which a plurality of microprotrusions are arranged, in which an average distance between adjacent microprotrusions is larger than 0.5 µm and 5.0 µm or smaller (Patent Document 5).

Antibiotics are used for purpose of treating human and livestock diseases, improving a productivity of agricultural, livestock, and marine products, storing foods, and the like, and are used as medicines, veterinary medicines, pesticides, feed additives, food additives, and the like.

In recent years, emergence of resistant bacteria which have resistance to multiple kinds of antibiotics and for which the antibiotics do not work or become difficult to work has become a problem. Therefore, use of antibiotics tends to be restricted for the purpose of suppressing the emergence of resistant bacteria.

For example, the followings have been proposed as a technique capable of exhibiting an antimicrobial effect without using antibiotics.
(6) A method for forming an anodic oxide coating on a surface of aluminum or an aluminum alloy, the method including performing an anodizing treatment under a predetermined condition, by using an aqueous solution containing 250 to 350 g/L of sulfuric acid, 15 to 25 g/L of nickel sulfate, and 80 to 320 g/L of a low-polymerized acrylic resin composition (Patent Document 6).
(7) A medical component made of a metal material, using a metal material as a base material, the medical component including, on a surface of the base material, a film having fine pores and/or fine unevenness, in which the fine pores and/or the fine unevenness are impregnated with iodine or an iodine compound (Patent Document 7).
(8) A method for surface treatment of aluminum or an aluminum alloy, the method including: forming an anodic oxide coating on a surface of aluminum or an aluminum alloy, by using a bath solution to which a low-weight acrylic resin is added; and further performing impregnating with germanium by using a bath solution containing organic germanium under a predetermined treatment condition (Patent Document 8).
(9) A method for surface treatment of aluminum or an alloy thereof, the method including: electrolyzing a base material formed of aluminum or an alloy thereof in an electrolytic solution in which one or two of silver nitrate and copper nitrate as metal nitrate or one or two of silver sulfate and copper sulfate as metal sulfate are added in a sulfuric acid bath, an oxalic acid bath, or a mixed bath thereof, by applying a current of AC/DC superimposition, PR that sends a negative wave, or a pulse wave that sends a negative wave, to form an anodic oxide coating on a surface of the base material, and at the same time, precipitate metal of the added nitrate or sulfate on the anodic oxide coating (Patent Document 9).
(10) An antimicrobial treatment method for an anodized aluminum material, the method including dispersing a titanium oxide TiO₂ fine powder in an electrolytic bath when an aluminum material is anodized in the electrolytic bath and a porous anodic oxide film is formed on the surface thereof (Patent Document 10).
(11) A synthetic polymer film having a surface having a plurality of projection portions, in which when viewed from the normal direction of the synthetic polymer film, a two-dimensional size of the plurality of projection portions is within a range of greater than 20 nm and smaller than 500 nm, the surface has a bactericidal effect, and a concentration of a nitrogen element contained in the surface is 0.7 atm% or higher (Patent Document 11).

### [Citation List]

### [Patent Document]

[Patent Document 1] Japanese Unexamined Patent Application, First Publication No. 2016-093939
[Patent Document 2] Japanese Unexamined Patent Application, First Publication No. 2016-104545
[Patent Document 3] Japanese Unexamined Patent Application, First Publication No. 2016-210164
[Patent Document 4] Japanese Unexamined Patent Application, First Publication No. 2016-215622
[Patent Document 5] Japanese Unexamined Patent Application, First Publication No. 2017-132916
[Patent Document 6] PCT International Publication No. WO 2004/067807
[Patent Document 7] PCT International Publication No. WO 2011/024216
[Patent Document 8] Japanese Unexamined Patent Application, First Publication No. 2010-001507
[Patent Document 9] Japanese Unexamined Patent Application, First Publication No. 2002-047596
[Patent Document 10] Japanese Unexamined Patent Application, First Publication No. 2000-064093
[Patent Document 11] Japanese Unexamined Patent Application, First Publication No. 2016-153510

### [Summary of Invention]

### [Technical Problem]

The antimicrobial articles and the like of (1) to (5) are produced by an imprint method using a mold having micropores, and the microprotrusions in the antimicrobial articles are made of a cured product of a curable resin. According to the study of the present inventors, in an antimicrobial article in which microprotrusions are made of a resin, there is a case where an antimicrobial effect may not be exhibited depending on kinds of the resin of the microprotrusions, an average distance between adjacent microprotrusions, kinds of bacteria, and the like.

Although the techniques described in (6) to (11) can exhibit an antimicrobial effect without using antibiotics, there is a case where a sufficient antimicrobial effect cannot be exhibited depending on kinds of bacteria and the like.

An object of the present invention is to provide an antimicrobial material capable of exhibiting an excellent antimicrobial effect without using a drug, a layered body having a layer made of such an antimicrobial material, a medical member having an antimicrobial material, an antimicrobial material production method, and an antimicrobial method, and is also to provide an antimicrobial layered body that exhibits a wider antimicrobial effect without using an antibiotic, and a production method thereof.

### [Solution to Problem]

[1] An antimicrobial layered body including:
   a non-metal substrate; and
   a metal oxide layer,
   in which the metal oxide layer is present on an outermost surface,
   the metal oxide layer contains an anion, and
   a total abundance ratio of at least one atom of a sulfur atom, a phosphorus atom, and a carbon atom which are derived from the anion is 1.0 atm% or more when analyzed by XPS.
[2] The antimicrobial layered body according to [1],
   in which the anion is selected from the group consisting of SO₄²⁻, PO₄³⁻, C₂O₄²⁻, C₃H₂O₄²⁻, C₄H₄O₅²⁻, and C₆H₅O₇³⁻.
[3] The antimicrobial layered body according to [1] or [2],
   in which, in the metal oxide layer present on the outermost surface, an abundance ratio of at least one atom of a sulfur atom, a phosphorus atom, and a carbon atom derived from the anion is 3.0 atm% or more.
[4] The antimicrobial layered body according to any one of [1] to [3], further including:
   a metal layer.
[5] The antimicrobial layered body according to any one of [1] to [4],
   in which a metal contained in the metal oxide layer and a metal layer is a valve metal.
[6] The antimicrobial layered body according to [5],
   in which the valve metal is aluminum.
[7] The antimicrobial layered body according to [5] or [6],
   in which, in the metal oxide layer present on the outermost surface, a total abundance ratio of the valve metal is 10 atm% or more when analyzed by XPS, and a total abundance ratio of a non-valve metal and a halogen atom is 1.0 atm% or less when analyzed by XPS.
[8] The antimicrobial layered body according to [7],
   in which the non-valve metal is at least one selected from the group consisting of silver, copper, titanium, and germanium, and
   the halogen atom is an iodine atom.
[9] The antimicrobial layered body according to any one of [1] to [8],
   in which, in the metal oxide layer present on the outermost surface, an atom derived from the anion is a sulfur atom and an abundance ratio of an oxygen atom is 45 atm% or more when analyzed by XPS.
[10] The antimicrobial layered body according to any one of [1] to [9],
   in which a total light transmittance of the metal oxide layer is 30% or more.
[11] The antimicrobial layered body according to any one of [1] to [10],
   in which a thickness of the metal oxide layer is 50 nm or larger and 10 µm or smaller.
[12] The antimicrobial layered body according to any one of [1] to [11],
   in which the metal oxide layer present on the outermost surface has a plurality of projection portions on the outermost surface thereof, and
   an average distance between adjacent projection portions is 20 to 600 nm.
[13] The antimicrobial layered body according to [12],
   in which the projection portion is a needle-like protrusion.
[14] An antimicrobial layered body production method which is for the antimicrobial layered body according to any one of [1] to [13], the method including:
   a step of anodizing a surface of a metal layer containing 95% by mass or more of a valve metal by using a polybasic acid having a concentration of 0.04 M or higher to form the metal oxide layer.
[15] The antimicrobial layered body production methodaccording to [14],
   in which a concentration of the polybasic acid is 0.3 M or higher.
[16] The antimicrobial layered body production method according to [14] or [15],
   in which the valve metal is aluminum, and
   the polybasic acid is sulfuric acid having a concentration of 3 M or higher.
[17] The antimicrobial layered body production method according to [16],
   in which a concentration of the sulfuric acid is 6 M or higher.
[18] An antimicrobial material including:
   an oxide film of a metal having a plurality of projection portions on a surface,
   in which an average distance between adjacent projection portions is 20 to 400 nm.
[19] The antimicrobial material according to [18],
   in which the projection portion is a needle-like protrusion.
[20] A layered body including:
   a layer made of the antimicrobial material according to [18] or [19].
[21] A medical member including:
   the antimicrobial material according to [18] or [19].
[22] An antimicrobial material production method for producing the antimicrobial material according to [18] or [19], the method including:
   forming an oxide film having a plurality of projection portions on a surface by performing, once or more times, a combination of a step of anodizing a metal base material to form an oxide film having a pore and a step of expanding a diameter of the pore.
[23] An antimicrobial method including:
   providing the antimicrobial material according to [18] or [19] at a place where growth of bacteria is desired to be suppressed.

### [Advantageous Effects of Invention]

An antimicrobial material of the present invention can exhibit an excellent antimicrobial effect without using a drug.

A layered body of the present invention can exhibit an excellent antimicrobial effect without using a drug.

A medical member of the present invention can exhibit an excellent antimicrobial effect without using a drug.

According to an antimicrobial material production method of the present invention, an antimicrobial material that can exhibit an excellent antimicrobial effect can be produced without using a drug.

According to an antimicrobial method of the present invention, an excellent antimicrobial effect can be exhibited without using a drug.

According to the present invention, it is possible to provide an antimicrobial layered body that exhibits a wider antimicrobial effect without using an antibiotic and a production method of the same.

### [Brief Description of Drawings]

Fig. 1 is a top view showing an example of an antimicrobial material of the present invention.
Fig. 2 is a sectional view taken along the line II-II of Fig. 1.
Fig. 3 is a sectional view showing a production step for the antimicrobial material of the present invention.
Fig. 4 is a sectional view showing an example of a layered body of the present invention.
Fig. 5 is a scanning electron microscope image of a surface of an oxide film of an antimicrobial material of Example 1.
Fig. 6 is a scanning electron microscope image of a section of the antimicrobial material of Example 1.
Fig. 7 is a sectional view showing an antimicrobial layered body production method of the present invention.
Fig. 8 is a scanning electron microscope image of a surface of an anodic oxide coating of an aluminum plate with an anodic oxide coating of Example 5.
Fig. 9 is a scanning electron microscope image of a section of the aluminum plate with an anodic oxide coating of Example 5.
Fig. 10 is a graph showing a total light transmittance of an antimicrobial layered body of Example 13.

### [Description of Embodiments]

It is assumed that a numerical range represented by "to" includes numerical values at both ends.

A "bacteria" means a germ, a fungus, and the like. Staphylococcus aureus, Escherichia coli, Bacillus subtilis, Lactic acid bacterium, Pseudomonas aeruginosa, Streptococcus, and the like are exemplary examples of the germ. Filamentous fungi (molds, mushrooms), yeast, and the like are exemplary examples of the fungus. Saccharomyces, Schizosaccharomyces, Cryptococcus, Candida, and the like are examples of a yeast.

"XPS" is an abbreviation for X-ray Photoelectron Spectroscopy.

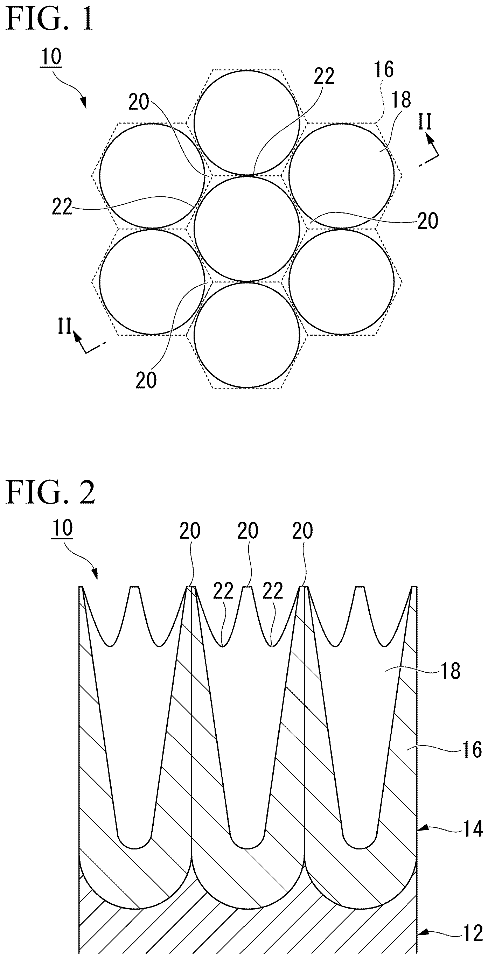

Dimensional ratios in Figs. 1 to 4 are different from actual ones for convenience of explanation. Further, in Figs. 2 to 4, the same components as those in Fig. 1 are designated by the same reference numerals, and a description thereof will not be repeated.

### [Antimicrobial material]

The antimicrobial material of the present invention includes an oxide film of a metal having a plurality of projection portions on a surface.

Fig. 1 is a top view showing an example of an antimicrobial material of the present invention, and Fig. 2 is a sectional view taken along the line II-II of Fig. 1. The antimicrobial material in a shown example is an example in which the metal is aluminum.

An antimicrobial material 10 has an aluminum base material 12 and an oxide film 14 formed on a surface of the aluminum base material 12.

A normal oxide film on aluminum is an assembly of a plurality of hexagonal columnar cells, and pores expanded in an axial direction of cells from the surface of the oxide film toward the aluminum base material are formed in the center of the cells.

In the oxide film 14 of the shown example, a pore of each cell 16 is expanded by a pore diameter expanding treatment to be described later to form an inverted conical recess portion 18. As the pore is expanded and the recess portion 18 is formed, in a surface of the oxide film 14 and the vicinity thereof, the oxide film 14 remains in a hexagonal lattice (broken line in the drawing) formed by boundaries of a plurality of cells 16 and in the vicinity thereof. At a lattice point of a hexagonal lattice at which three cells 16 contact with one another and in the vicinity thereof, the oxide film 14 is not eroded into a recess portion 18, and a projection portion 20 of the needle-like protrusion is formed. In a lattice line of a hexagonal lattice where two cells 16 are in contact with each other and in the vicinity thereof, a ridge portion 22 having the oxide film 14 eroded by the recess portion 18 and having a height lower than that of the projection portion 20 is formed so as to connect adjacent projection portions 20.

The metal may be any metal that can form an oxide film having pores by anodizing. Aluminum, niobium, tantalum, tungsten, titanium, zirconium, hafnium, an alloy made of two or more of these metals, an alloy of one or more of these metals with another metal, and the like are exemplary examples of the metal. As the metal, the aluminum or an alloy thereof is preferable in that it is easy to form an oxide film having a plurality of projection portions.

A deposition film, a foil, a plate, and a formed product other than these are exemplary examples of a form of the metal base material.

An average distance between adjacent projection portions is 20 to 400 nm, preferably 25 to 350 nm, and more preferably 30 to 300 nm. When the average distance between adjacent projection portions is equal to or greater than a lower limit value of the above range, an oxide film having a plurality of projection portions is likely to be formed. When the average distance between adjacent projection portions is equal to or smaller than an upper limit value of the range, an antimicrobial effect is exhibited.

The "average distance between adjacent projection portions" is an average of values obtained by measuring a distance from the center of a top portion of the projection portion to the center of a top portion of the adjacent projection portion at 50 points, by an electron microscope observation.

An average height of projection portion is preferably 50 to 2,500 nm, and more preferably 70 to 2,000 nm.

An "average height of the projection portions" is an average of values obtained by measuring a height difference between a top portion of the projection portion and a bottom portion of the recess portion present between the projection portions at 50 points, by an electron microscope observation.

The projection portion is preferably a needle-like protrusion in that an antimicrobial effect is further improved.

The needle-like protrusion refers to a projection portion having an aspect ratio of 1.5 or more obtained by dividing the average height of the projection portion by the average distance between adjacent projection portions.

The aspect ratio (average height/average distance) of the projection portions is preferably 1.5 to 10, more preferably 2 to 8, and even more preferably 3 to 7. When the aspect ratio of the projection portion is equal to or higher than the lower limit value of the range, the antimicrobial effect is further improved. When the aspect ratio of the projection portion is equal to or less than the upper limit value of the range, a durability of the projection portion is good.

The oxide film preferably has a ridge portion having a height lower than that of the projection portion, which is formed so as to connect the adjacent projection portions. Since the oxide film has the ridge portion connecting the adjacent projection portions, the projection portion is reinforced by the ridge portion, and the durability of the projection portion is further improved.

Since the antimicrobial material of the present invention described above has a plurality of projection portions having an average distance of 20 to 400 nm between adjacent projection portions on the surface, the antimicrobial effect can be exhibited. Further, since the projection portion is formed of an oxide film of a metal, an excellent antimicrobial effect can be reliably exhibited as compared with the projection portion formed of a cured product of a conventional curable resin. Although it is not clear why the projection portion formed of the oxide film of the metal can exhibit an excellent antimicrobial effect as compared with the projection portion formed of the cured product of the curable resin, it is considered that the metal oxide film is harder than the cured product of the curable resin, and the hardness affect the expression of the antimicrobial effect.

### [Antimicrobial material production method]

An antimicrobial material production method of the present invention is a method for forming an oxide film having a plurality of projection portions on a surface by performing, once or more times, a combination of a step of anodizing a metal base material to form an oxide film having a pore and a step of expanding a diameter of the pore.

Hereinafter, the antimicrobial material production method of the present invention will be described in detail, taking as an example a case where the oxide film is an aluminum oxide film (Alumite).

The antimicrobial material having an aluminum oxide film on the surface can be produced, for example, through the following steps (a) to (f). Although the regularity of the arrangement of the pores is slightly reduced, the steps (b) and (c) may not be performed, and the steps (d) and (e) may be repeated after the step (a), or the step (d) may be performed only once after the step (a).
(a) A step of anodizing an aluminum base material in an electrolytic solution to form an oxide film.
(b) A step of removing the oxide film and forming an anodized pore generation point.
(c) A step of anodizing the aluminum base material again in the electrolytic solution to form an oxide film having pores at pore generation points.
(d) A step of expanding the diameter of the pores.
(e) A step of anodizing again in the electrolytic solution after the step (d).
(f) A step of repeating steps (d) and (e).

### Step (a):

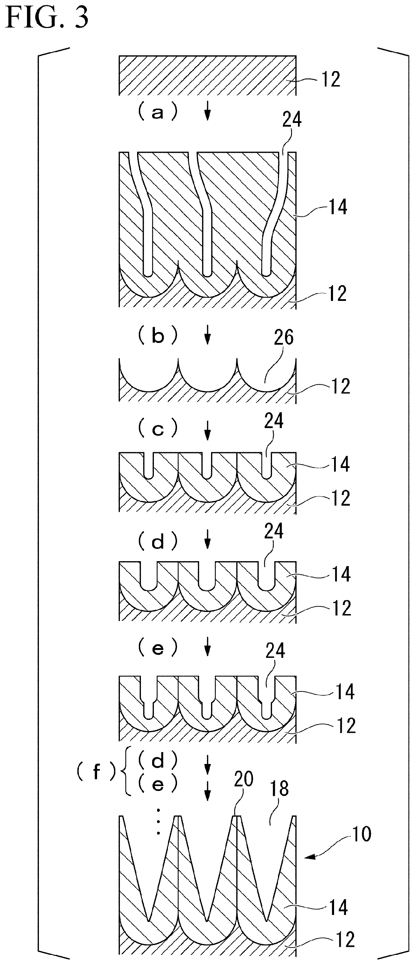

As shown in Fig. 3, when the aluminum base material 12 is anodized, an oxide film 14 having a pore 24 is formed. A regularity of the pores formed by anodization is extremely low in an initial stage, but the regularity of the pores is improved by long-term anodizing. The anodizing time is preferably 5 minutes or longer, and more preferably 15 minutes or longer. However, although the regularity of the pores is improved by performing anodization for a long time, the pores tend to be relatively deep, so that a treatment of the step (b) is performed and used as a generation point for forming regular pores. If only pores are formed without expecting regularity, the treatment time until the desired pore depth is reached may be appropriately set.

A purity of aluminum is preferably 99% or higher, more preferably 99.5% or higher, and particularly preferably 99.8% or higher. When the purity of aluminum is low, the regularity of the pores obtained by anodizing may decrease.

Sulfuric acid, an oxalic acid aqueous solution, and a phosphoric acid aqueous solution are exemplary examples of the electrolytic solution.

In a case where sulfuric acid is used as the electrolytic solution:
a concentration of the sulfuric acid is preferably 0.7 mol/L or lower. When the concentration of the sulfuric acid exceeds 0.7 mol/L, a current value may become too high and a constant voltage may not be maintained.

When a formation voltage is 25 to 30 V, an oxide film having pores with high regularity with 63 nm of an interval between adjacent pores can be formed.

A temperature of the electrolytic solution is preferably 30°C or lower, and more preferably 20°C or lower.

In a case where oxalic acid aqueous solution is used as the electrolytic solution:
a concentration of the oxalic acid is preferably 0.7 mol/L or lower. When the concentration of the oxalic acid exceeds 0.7 mol/L, a current value may become too high and a surface of the oxide film may become rough.

When a formation voltage is 30 to 100 V, an oxide film having pores with high regularity with 100 to 200 nm of an interval between adjacent pores can be formed.

A temperature of the electrolytic solution is preferably 60°C or lower, and more preferably 45°C or lower.

In a case where phosphoric acid aqueous solution is used as the electrolytic solution:
a concentration of the phosphoric acid is preferably 2.5 mol/L or lower. When the concentration of the oxalic acid exceeds 2.5 mol/L, a current value may become too high and pores may be broken.

When a formation voltage is 180 to 250 V, an oxide film having pores with high regularity with 500 nm of an interval between adjacent pores can be formed.

A temperature of the electrolytic solution is preferably 60°C or lower, and more preferably 45°C or lower.

### Step (b):

As shown in Fig. 3, the regularity of the pores can be improved by temporarily removing the oxide film 14 and setting a depression on the surface of the aluminum base material 12 as an anodized pore generation point 26.

A method in which aluminum is not dissolved but is dissolved in a solution that selectively dissolves the oxide film to remove the oxide film is an exemplary example of the method for removing the oxide film. A chromic acid/phosphoric acid mixed solution is an exemplary example of such a solution.

### Step (c):

As shown in Fig. 3, when the aluminum base material 12 from which the oxide film has been removed is anodized again, an oxide film 14 having a columnar pore 24 is formed.

The anodizing may be carried out under the same conditions as in the step (a). The longer the anodizing time, the deeper the pores can be obtained.

### Step (d):

As shown in Fig. 3, a treatment of expanding the diameter of the pore 24 (hereinafter, referred to as a pore diameter expanding treatment) is performed. The pore diameter expanding treatment is a treatment for expanding the diameter of the pores obtained by anodizing by immersing in a solution that dissolves the oxide film. A phosphoric acid aqueous solution of about 5% by mass is an exemplary example of such a solution.

The longer the pore diameter expanding treatment time, the larger the pore diameter.

### Step (e):

As shown in Fig. 3, when anodized again, the columnar pore 24 having a small diameter extending downward from the bottom portion of the columnar pore 24 is further formed.

The anodizing may be carried out under the same conditions as in the step (a). The longer the anodizing time, the deeper the pores can be obtained.

### Step (f):

As shown in Fig. 3, when the pore diameter expanding treatment in the step (d) and the anodizing in the step (e) are repeated, the antimicrobial material 10 having an oxide film 14 in which a recess portion 18 having a shape in which a diameter continuously decreases from the opening portion in the depth direction and the projection portion 20 surrounded by three recess portions 18 are formed can be obtained. It is preferable that the treatment ends with step (d) as a final step.

In order to form an oxide film having a plurality of projection portions on the surface, the number of repetitions of the step (d) and the step (e) is increased, the time for the pore diameter expanding treatment in the step (d) is lengthened, and the like. Accordingly, the diameter of the pores may be expanded until the ridge portion is formed on the oxide film.

The average distance between adjacent projection portions is determined by a distance between adjacent pores in the oxide film formed by anodizing. The distance between adjacent pores tends to decrease as the anodizing formation voltage decreases and to increase as the formation voltage increases.

### [Layered body]

A layered body of the present invention has a layer made of the antimicrobial material of the present invention.

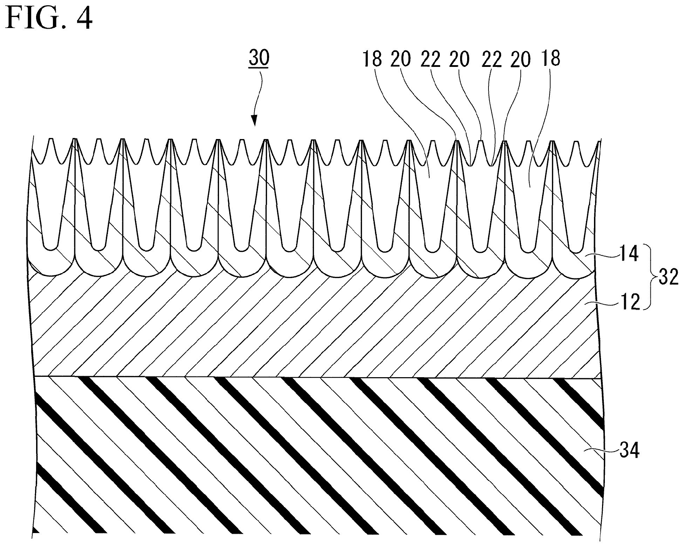

Fig. 4 is a sectional view showing an example of a layered body of the present invention. The layered body in a shown example is an example in which the metal in the antimicrobial material is aluminum.

The layered body 30 has a layer 32 made of the antimicrobial material and the other base material 34.

The layer 32 made of the antimicrobial material has an aluminum base material 12 in contact with the other base material 34 and the oxide film 14 formed on the surface of the aluminum base material 12.

The oxide film 14 has a plurality of recess portions 18, projection portions 20 surrounded by the recess portions 18, and the ridge portion 22 having a height lower than that of the projection portion 20, which is formed so as to connect the adjacent projection portions 20.

A cured product of a curable resin, plastic, glass, ceramics, metal, and the like are exemplary examples of the material of the other base material.

A film, a sheet, a plate, and a formed product other than these are exemplary examples of the other base material.

The layered body of the present invention can be produced, for example, by producing a layered body having the metal base material and the other base material, and by performing, once or more times, a combination of a step of anodizing a metal base material of the layered body to form an oxide film having a pore and a step of expanding a diameter of the pore.

Specifically, in a case where the layered body of the present invention is an aluminum-deposited film, a combination of a step of anodizing a deposition film of the aluminum-deposited film to form an oxide film having a pore and a step of expanding a diameter of the pore is performed once or more times.

The layered body of the present invention may be any one having a metal oxide film having a plurality of projection portions on the outermost surface, and is not limited to the layered body 30 of the shown example.

For example, the metal base material may be completely anodized to form a layered body having the oxide film 14 and the other base material 34 in contact with the oxide film 14. In a case where the other base material is a transparent plastic film and the metal base material is a deposition film of aluminum, an antimicrobial transparent barrier film in which an alumina layer having a plurality of projection portions is formed on the surface of the plastic film is obtained by completely anodizing the deposition film.

### [Application of antimicrobial material]

The antimicrobial material of the present invention is provided, for example, at a place where the growth of bacteria is desired to be suppressed.

The followings are exemplary examples of applications of the antimicrobial material and the layered body of the present invention.

Medical member: details of the medical member will be described later.

Filter: an air purifier filter, an air conditioner filter, an air filter, and the like.

Water treatment member: a water purifier, a shower nozzle, an inner surface of a pipe, and the like.

Building material: an interior material (such as wallpaper, a wall material, a floor material, a ceiling material, a door surface material, and counters), a water supply, a wind film, an exterior material, a handrail, and the like.

Packaging material: a food packaging film (such as an aluminum-deposited film and a barrier film), a container, a bottle, and the like.

Home appliance part: a touch panel, a display front material, a humidifier tank, a washing tubs of a washing machine, and the like.

Furniture: a table, a chair, a cooking utensil, and the like.

Household product: a mold prevention material for closet, an attic mold prevention material, and the like.

Vehicle part: an interior material, a strap, a handrail, and the like.

Agricultural material: a vinyl greenhouse, a hydroponic cultivation facility, a piping, and the like.

### [Medical member]

The medical member of the present invention has the antimicrobial material of the present invention.

The medical member of the present invention may be made of the antimicrobial material of the present invention, or may be a layered body having a layer made of the antimicrobial material of the present invention, and may be a combination of the antimicrobial material or layered body of the present invention and another member.

The medical member of the present invention may be an artificial organ or a medical device itself, may be a part of an artificial organ, a medical device, medical equipment, or the like, may be a part of a medical facility, or may be a packaging material for an artificial organ, a medical device, medical equipment, and the like.

A dental implant (artificial teeth), artificial heart, an artificial joint, and the like are exemplary examples of the artificial organ.

A surgical instrument (such as a scalpel, scissors, forceps, tweezers, a retractor, catheters, a stent, and a fixing bolt), a syringe, a stethoscope, a consultant, a microscope, a cradle, a dental device (such as dental scaler and dental mirror), and the like are exemplary examples of the medical device.

An operating table, an artificial dialyzer, an infusion pump, a heart-lung machine, a dialysate supply device, a component blood sampling device, a ventilator, an X-ray imaging device, an electrocardiograph, an ultrasonic diagnostic device, a particle beam therapy device, an analyzer, a pacemaker, a hearing aid, a massager, and the like are exemplary examples of the medical equipment.

An interior material (such as wallpaper, a wall material, a floor material, a ceiling material, a door surface material, and a counter) for a hospital room, an operating room, a bathroom, a toilet, and the like, a handrail, a doorknob, and the like are exemplary examples of the medical facility.

### [Antimicrobial layered body]

The antimicrobial layered body of the present invention includes a non-metal substrate; and
a metal oxide layer, in which the metal oxide layer is present on an outermost surface,
the metal oxide layer contains an anion, and
a total abundance ratio of at least one atom of a sulfur atom, a phosphorus atom, and a carbon atom which are derived from the anion is 1.0 atm% or more when analyzed by XPS.

The antimicrobial layered body of the present invention does not exhibit an antimicrobial property only by supporting Ag, Cu, Zn, or the like on anodized porous alumina. In the antimicrobial layered body of the present invention, the metal is anodized with a high-concentration electrolytic solution and the anion is doped into the coating, and the metal itself exhibits an antimicrobial property.

### <Non-metal substrate>

The non-metal substrate is not particularly limited as long as the substrate is made of non-metal, and the non-metal substrate is, for example, a resin or glass.

Specific examples of the resin include polypropylene, polyethylene, polyethylene terephthalate, polystyrene, and nylon, but are not limited thereto.

### <Metal oxide layer>

The metal oxide layer is present on the outermost surface of the antimicrobial layered body of the present invention.

The metal oxide layer contains an anion.

The metal contained in the metal oxide layer is not particularly limited, but a valve metal is preferable.

The valve metal is a metal that forms a passivation oxide film on a surface by contact with an oxidizing acid or an oxidation treatment such as anodizing treatment. Specific examples of the valve metal include aluminum, chromium, titanium, and an alloy of two or more kinds thereof, but are not limited thereto.

As the valve metal, aluminum is preferable in that aluminum has good workability, high safety, and low cost.

Metals other than the valve metal are called "non-valve metal".

A total abundance ratio of the valve metal in the metal oxide layer is 10 atm% or more, preferably 15 atm% or more, and more preferably 20 atm% or more when analyzed by XPS. An upper limit is not particularly limited, and is usually 40 atm%.

An analysis method and an analysis condition using XPS are as follows.
X-ray photoelectron spectroscopy analyzer: Quantum-2000 manufactured by ULVAC-PHI
X-ray source: Monochromated-A1-Kα ray (output 16kV 34W)
Extraction angle: 45°
Measurement area: 300 µm

The total abundance ratio of the non-valve metal and the halogen atom in the metal oxide layer is preferably 1.0 atm% or less when analyzed by XPS. A lower limit is not particularly limited, but is usually 0.0 atm%. The antimicrobial layered body according to the present invention does not exhibit an antimicrobial property only by supporting an antimicrobial material such as silver, copper, titanium oxide, or iodine on the outermost surface, but the oxide film itself exhibits an antimicrobial property.

The non-valve metal is other than the metal contained in the metal oxide layer, and silver, copper, titanium, and germanium are specific examples thereof.

The halogen atom is preferably at least one selected from the group consisting of a fluorine atom, a chlorine atom, a bromine atom, and an iodine atom, more preferably at least one selected from the group consisting of a chlorine atom, a bromine atom, and an iodine atom, and further preferably an iodine atom.

The analysis method and the analysis condition using XPS are the same as the analysis method and analysis condition for the total abundance ratio of the valve metal.

In the metal oxide layer, the total abundance ratio of at least one atom of a sulfur atom, a phosphorus atom, and a carbon atom derived from the anion contained in the metal oxide layer is 1.0 atm% or more, preferably 2.5 atm% or more, and more preferably 3.0 atm% or more when analyzed by XPS (X-ray photoelectron spectroscopy) from the viewpoint that a strong antimicrobial property can be exhibited. An upper limit is not particularly limited, but is usually 10 atm%.

In order to set the total abundance ratio of at least one atom of a sulfur atom, a phosphorus atom, and a carbon atom derived from the anion contained in the metal oxide layer within the preferable range, the metal oxide layer may be produced, for example, by [Antimicrobial layered body production method] to be described later. In particular, by adjusting kinds and concentration of the polybasic acid, the total abundance ratio of a sulfur atom, a phosphorus atom, and a carbon atom derived from the anion can be adjusted.

In XPS, the abundance ratio (atm%) of each element on the surface can be determined from a peak intensity of the wide spectrum.

The at least one atom of the sulfur atom, the phosphorus atom, and the carbon atom derived from the anion is preferably the sulfur atom. In this case, the abundance ratio of an oxygen atom in the metal oxide layer is preferably 45 atm% or more, and more preferably 55 atm% or more when analyzed by XPS. An upper limit is not particularly limited, but is usually 60 atm%.

The analysis method and the analysis condition using XPS are the same as the analysis method and analysis condition for the total abundance ratio of the valve metal.

Whether or not at least one atom of the sulfur atom, the phosphorus atom, and the carbon atom is derived from an anion can be determined by chemical shift in XPS.

For example, when a peak appears at 169.8 ± 1.4, it can be determined as the sulfur atom derived from an anion. In addition, when a peak appears at 290 ± 1.3, it can be determined as the carbon atom derived from an anion, and when a peak appears at 132.5 ± 0.4, it can be determined as a phosphorus atom derived from an anion.

The anion is preferably one or more selected from the group consisting of a sulfate ion (SO₄²⁻), a phosphate ion (PO₄³⁻), an oxalate ion (C₂O₄²⁻), a malonate ion (C₃H₂O₄²⁻), a malate ion (C₄H₄O₅²⁻), and a citrate ion (C₆H₅O₇³⁻). Among these, the sulfate ion or the oxalate ion is more preferable, and sulfate ion is particularly preferable, from the viewpoint of exhibiting a strong antimicrobial property.

In the antimicrobial layered body of the present invention, the total light transmittance of the metal oxide layer is preferably 30% or more, more preferably 50% or more, and further preferably 60% or more. An upper limit is not particularly limited, but is usually 95%. When the total light transmittance of the metal oxide layer is 50% or more, the material of the present invention can be suitably used in applications for which transparency in which it is necessary to visually recognize what is inside is required.

Here, the total light transmittance of the metal oxide layer can be measured by a known method of the related art with reference to JIS K 7136: 2000 "Plastics - Determination of haze for transparent materials" and the like.

In the antimicrobial layered body of the present invention, a thickness of the metal oxide layer is preferably 50 nm or larger and 10 µm or smaller, more preferably 55 nm or larger and 1 µm or smaller, and further preferably 60 nm or larger and 500 nm or smaller. When the thickness of the metal oxide layer is within the range, it is possible to prevent the anodizing treatment time to be described later, from becoming long.

Here, the thickness of the metal oxide layer can be confirmed by observing a section with an SEM or the like.

In the antimicrobial layered body of the present invention, the metal oxide layer may have a plurality of projection portions on the outermost surface thereof. In this case, the average distance between the adjacent projection portions may be in the range of 20 to 600 nm. A function such as hydrophilicity or water repellency can be further imparted by providing a structure in the range on the surface.

The projection portion is preferably a needle-like protrusion.

### <Metal layer>

The antimicrobial layered body may further have a metal layer. The metal contained in the metal layer may be the same as the metal contained in the metal oxide layer. In addition, in a case where the metal layer deposited on a resin film or the like is anodized, various metal thin films having a thickness of several nm may be provided in order to improve the adhesion between the metal layer and the resin film.

### <Using method and application of antimicrobial layered body>

A using method of the antimicrobial layered body of the present invention is not particularly limited, but it is preferable to provide the antimicrobial layered body of the present invention at a place where the growth of bacteria is desired to be suppressed.

The followings are exemplary examples of applications of the antimicrobial layered body of the present invention.

Medical member: details of the medical member will be described later.

Filter: an air purifier filter, an air conditioner filter, an air filter, and the like.

Water treatment member: a water purifier, a shower nozzle, an inner surface of a pipe, and the like.

Building material: an interior material (such as wallpaper, a wall material, a floor material, a ceiling material, a door surface material, and counters), a water supply, a wind film, an exterior material, a handrail, and the like.

Packaging material: a food packaging film (such as an aluminum-deposited film and a barrier film), a container, a bottle, and the like.

Home appliance part: a touch panel, a display front material, a humidifier tank, a washing tubs of a washing machine, and the like.

Furniture: a table, a chair, a cooking utensil, and the like.

Household product: a mold prevention material for closet, an attic mold prevention material, and the like.

Vehicle part: an interior material, a strap, a handrail, and the like.

Agricultural material: a vinyl greenhouse, a hydroponic cultivation facility, a piping, and the like.

The medical member may be made of the antimicrobial layered body of the present invention, or may be a combination of the antimicrobial layered body of the present invention and another member.

The medical member may be an artificial organ or a medical device itself, may be a part of an artificial organ, a medical device, medical equipment, or the like, may be a part of a medical facility, or may be a packaging material for an artificial organ, a medical device, medical equipment, and the like.

A dental implant (artificial teeth), artificial heart, an artificial joint, and the like are exemplary examples of the artificial organ.

A surgical instrument (such as a scalpel, scissors, forceps, tweezers, a retractor, catheters, a stent, and a fixing bolt), a syringe, a stethoscope, a consultant, a microscope, a cradle, a dental device (such as dental scaler and dental mirror), and the like are exemplary examples of the medical device.

An operating table, an artificial dialyzer, an infusion pump, a heart-lung machine, a dialysate supply device, a component blood sampling device, a ventilator, an X-ray imaging device, an electrocardiograph, an ultrasonic diagnostic device, a particle beam therapy device, an analyzer, a pacemaker, a hearing aid, a massager, and the like are exemplary examples of the medical equipment.

An interior material (such as wallpaper, a wall material, a floor material, a ceiling material, a door surface material, and a counter) for a hospital room, an operating room, a bathroom, a toilet, and the like, a handrail, a doorknob, and the like are exemplary examples of the medical facility.

### [Antimicrobial layered body production method]

An antimicrobial layered body production method of the present invention (hereinafter, may be simply referred to as the "production method of the present invention") includes a step of anodizing a surface of a metal layer containing 95% by mass or more of a valve metal by using a polybasic acid having a concentration of 0.04 M or higher to form the metal oxide layer.

The valve metal is as described above.

The metal layer contains 95% by mass or more of the valve metal, which means that the purity of the valve metal is 95% by mass or more. The purity of the valve metal is not particularly limited as long as the purity is 95% by mass or more, but is preferably 99% by mass or more, more preferably 99.9% by mass or more, and further preferably 99.99% by mass or more. When the purity of the valve metal is 95% by mass, it is possible to prevent dissimilar metals from falling off during anodizing and a macroscopic defect from occurring on the surface.

A polybasic acid is an acid having a basicity of 2 or more. Here, the basicity is the number of hydrogen atoms substituted with metal atoms among the hydrogen atoms contained in one molecule of the acid.

The polybasic acid is not particularly limited, and sulfuric acid (dibasic acid), phosphoric acid (tribasic acid), oxalic acid (dibasic acid), malonic acid (dibasic acid), and malic acid (dibasic acid), and citric acid (tribasic acid) are exemplary examples thereof.

The polybasic acid is preferably at least one selected from the group consisting of sulfuric acid, phosphoric acid, oxalic acid, malonic acid, malic acid, and citric acid, more preferably at least one selected from the group consisting of sulfuric acid, phosphoric acid, and oxalic acid, and further preferably sulfuric acid.

In the production method of the present invention, one kind of the polybasic acid may be used, or two or more kinds thereof may be used.

A concentration of the polybasic acid is not particularly limited as long as the concentration is 0.04 M (0.04 mol/dm³) or higher, and is preferably 0.3 M (0.3 mol/dm³) or higher, and more preferably 3 M (3 mol/dm³) or higher. An upper limit is not particularly limited, but in a case where the concentration exhibits an oxidizing power, a concentration not exhibiting oxidizing power is preferable.

In a case where the valve metal is aluminum and the polybasic acid is sulfuric acid, the concentration of the sulfuric acid is preferably 3 M (0.3 mol/dm³) or higher, and more preferably 6 M (6 mol/dm³) or higher. An upper limit of the concentration is usually 15 M (15 mol/dm³) or lower, and preferably 12 M (12 mol/dm³) or lower.

In the production method of the present invention, the anodizing is not limited to once, but may be performed twice or more. When performing the anodizing twice or more, a kind and a concentration of the polybasic acid may be changed.

Hereinafter, the production method of the present invention will be specifically described by taking, as an example, the case where aluminum is used as the valve metal.

The antimicrobial layered body of the present invention can be produced, for example, through the following treatments of (a) and (b).
(a) Anodizing an aluminum base material in an electrolytic solution to form an oxide film
(b) Attaching the anodized aluminum base material to non-metal base material

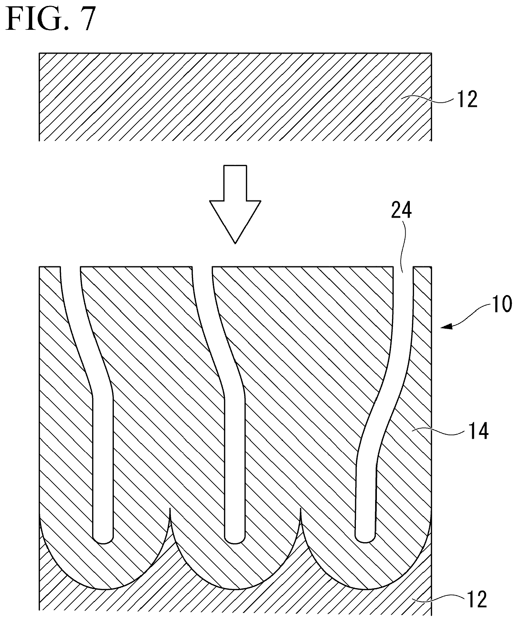

The treatment of (a) will be described with reference to Fig. 7.

As shown in Fig. 7, when an aluminum base material 112 is anodized, an oxide film 114 having a pore 124 is formed to obtain an aluminum plate 110 with an anodic oxide coating. The regularity of the pores formed by anodizing improves as the anodizing takes longer. However, since the antimicrobial property of the antimicrobial layered body of the present invention is not affected by the regularity of the pores, it is not necessary to perform anodizing for a long time.

A purity of aluminum is preferably 95% or higher, more preferably 99% or higher, further preferably 99.5% or higher, and still further preferably 99.9% or higher. The higher the purity of aluminum, the shorter the time required for anodizing may be possible.

The electrolytic solution is preferably sulfuric acid, an oxalic acid aqueous solution, or a phosphoric acid aqueous solution.

In a case where the sulfuric acid is used as the electrolytic solution, a concentration of the sulfuric acid is 0.3 M or higher, preferably 3 M or higher, and more preferably 3 to 4.5 M. Energizing time during anodizing is preferably 30 seconds to 15 minutes, more preferably 1 to 10 minutes, and further preferably 1 to 5 minutes. An applied voltage during anodizing is preferably 15 to 50 V, and more preferably 20 to 30 V. A temperature of the electrolytic solution during anodizing is preferably 0°C to 30°C, and more preferably 0°C to 20°C.

In a case where the oxalic acid aqueous solution is used as the electrolytic solution, a concentration of oxalic acid is preferably 0.01 M or higher, more preferably 0.01 to 0.7 M, and further preferably 0.01 to 0.1 M. Energizing time during anodizing is preferably 30 seconds to 15 minutes, more preferably 1 to 10 minutes, and further preferably 1 to 5 minutes. An applied voltage during anodizing is preferably 50 to 100 V, and more preferably 60 to 100 V. A temperature of the electrolytic solution during anodizing is preferably 0°C to 30°C, and more preferably 0°C to 20°C.

In a case where a phosphoric acid aqueous solution is used as the electrolytic solution, a concentration of the phosphoric acid is preferably 0.01 M or more, more preferably 0.01 to 2.5 M, and further preferably 0.05 to 1 M. Energizing time during anodizing is preferably 1 to 15 minutes, more preferably 1 to 10 minutes, and further preferably 5 to 10 minutes. An applied voltage during anodizing is preferably 100 to 300 V, and more preferably 150 to 250 V. A temperature of the electrolytic solution during anodizing is preferably 0°C to 20°C, and more preferably 0°C to 10°C.

An antimicrobial layered body can be obtained by the treatment of (b). As the non-metal base material used in the present treatment, it is preferable to use a resin substrate made of polyethylene, polypropylene, polyethylene terephthalate, nylon, polystyrene or the like, or a layered body thereof.

The anodizing treatment of (a) may be performed after attaching the aluminum base material to the non-metal base material without performing the treatment of (b). Also, an aluminum film formed on a non-metal base material by a method such as sputtering or vapor deposition may be used as an aluminum base material to be subjected to the anodizing treatment of (a). As the non-metal base material, it is preferable to use a resin substrate made of polyethylene, polypropylene, polyethylene terephthalate, nylon, polystyrene or the like, or a layered body thereof.

In addition, the metal oxide layer can be made transparent by completely anodizing the aluminum base material by the anodizing treatment of (a). In this case, in order to make the antimicrobial layered body transparent, it is preferable to use a translucent resin substrate as a resin substrate. The aluminum base material is preferably a thin film in order to reduce the time required for the step of completely anodizing the aluminum base material. Specifically, a thickness of the aluminum base material is preferably 50 nm or larger and 1 µm or smaller, and more preferably 55 nm or larger and 500 nm or smaller.

### [Examples]

Hereinafter, the present invention will be described more specifically with reference to examples, but the present invention is not limited thereto. Embodiments of the present invention can be variously modified within the range not changing the gist of the present invention.

### [Example 1]

An aluminum plate having a purity of 99.99% was polished by padding and electropolished in a perchloric acid/ethanol mixed solution (1/4 volume ratio) to be mirror-finished.

### Step (a):

The electropolished aluminum plate was anodized in a 0.3 mol/L oxalic acid aqueous solution for 60 minutes under the conditions of DC 40 V and a temperature of 17°C.

### Step (b):

The aluminum plate on which an oxide film was formed was immersed in a 6% by mass phosphoric acid/1.8% by mass chromic acid mixed aqueous solution for 6 hours to remove a part or an entirety of the oxide film.

### Step (c):

The aluminum plate from which the oxide film was removed was anodized in a 0.3 mol/L oxalic acid aqueous solution for 30 seconds under the conditions of DC 40 V and a temperature of 17°C.

### Step (d):

The aluminum plate on which the oxide film was formed was immersed in 5% by mass phosphoric acid at 30°C for 11 minutes to expand a pore diameter.

### Step (e):

The aluminum plate which was subjected to the pore diameter expanding treatment was anodized in a 0.3 mol/L oxalic acid aqueous solution for 30 seconds under the conditions of DC 40 V and a temperature of 17°C.

### Step (f):

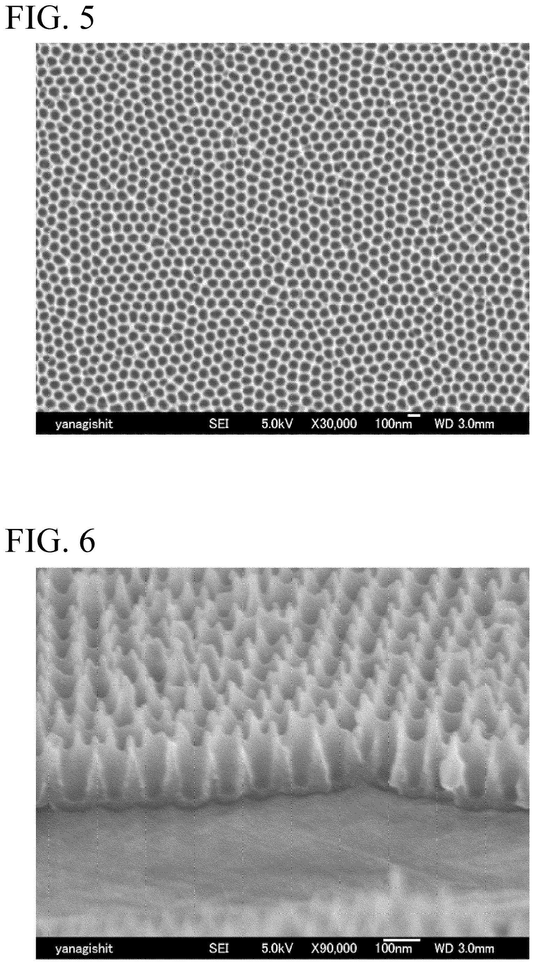

The step (d) and the step (e) are repeated a total of four times, and finally the step (d) is performed to obtain an antimicrobial material (test product) on which an oxide film (alumite) having a substantially conical recess portion with an average distance of 100 nm and a projection portion surrounded by the recess portion was formed. Table 1 shows an average distance, an average height, and an aspect ratio of the projection portions. In addition, Fig. 5 shows a scanning electron microscope image of a surface of the oxide film of the antimicrobial material, and Fig. 6 shows a scanning electron microscope image of a section of the antimicrobial material.

### [Example 2]

An aluminum plate having a purity of 99.99% was polished by padding and electropolished in a perchloric acid/ethanol mixed solution (1/4 volume ratio) to be mirror-finished.

### Step (a):

The electropolished aluminum plate was anodized in a 0.3 mol/L sulfuric acid for 30 minutes under the conditions of DC 25 V and a temperature of 17°C.

### Step (b):

The aluminum plate on which an oxide film was formed was immersed in a 6% by mass phosphoric acid/1.8% by mass chromic acid mixed aqueous solution for 6 hours to remove a part or an entirety of the oxide film.

### Step (c):

The aluminum plate from which the oxide film was removed was anodized in a 0.3 mol/L sulfuric acid for 6 seconds under the conditions of DC 25 V and a temperature of 17°C.

### Step (d):

The aluminum plate on which the oxide film was formed was immersed in 10% by mass phosphoric acid at 30°C for 4 minutes to expand a pore diameter.

### Step (e):

The aluminum plate which was subjected to the pore diameter expanding treatment was anodized in a 0.3 mol/L sulfuric acid for 6 seconds under the conditions of DC 25 V and a temperature of 17°C.

### Step (f):

The step (d) and the step (e) are repeated a total of five times to obtain an antimicrobial material (test product) on which an oxide film (alumite) having a substantially conical recess portion with an average distance of 63 nm and a projection portion surrounded by the recess portion was formed. Table 1 shows an average distance, an average height, and an aspect ratio of the projection portions.

### [Example 3]

An aluminum plate having a purity of 99.99% was polished by padding and electropolished in a perchloric acid/ethanol mixed solution (1/4 volume ratio) to be mirror-finished.

### Step (a):

The electropolished aluminum plate was anodized in a 0.05 mol/L oxalic acid aqueous solution for 30 minutes under the conditions of DC 80 V and a temperature of 17°C.

### Step (b):

The aluminum plate on which an oxide film was formed was immersed in a 6% by mass phosphoric acid/1.8% by mass chromic acid mixed aqueous solution for 6 hours to remove a part or an entirety of the oxide film.

### Step (c):

The aluminum plate from which the oxide film was removed was anodized in a 0.05 mol/L oxalic acid aqueous solution for 15 seconds under the conditions of DC 80 V and a temperature of 17°C.

### Step (d):

The aluminum plate on which the oxide film was formed was immersed in 5% by mass phosphoric acid at 30°C for 15 minutes to expand a pore diameter.

### Step (e):

The aluminum plate which was subjected to the pore diameter expanding treatment was anodized in a 0.05 mol/L oxalic acid aqueous solution for 15 seconds under the conditions of DC 80 V and a temperature of 17°C.

### Step (f):

The step (d) and the step (e) are repeated a total of five times to obtain an antimicrobial material (test product) on which an oxide film (alumite) having a substantially conical recess portion with an average distance of 200 nm and a projection portion surrounded by the recess portion was formed. Table 1 shows an average distance, an average height, and an aspect ratio of the projection portions.

### [Example 4]

An aluminum plate having a purity of 99.99% was polished by padding and electropolished in a perchloric acid/ethanol mixed solution (1/4 volume ratio) to be mirror-finished.

### Step (a):

The electropolished aluminum plate was anodized in a 0.1 mol/L phosphoric acid aqueous solution for 60 minutes under the conditions of DC 195 V and a temperature of 0°C.

### Step (b):

The aluminum plate on which an oxide film was formed was immersed in a 6% by mass phosphoric acid/1.8% by mass chromic acid mixed aqueous solution for 6 hours to remove a part or an entirety of the oxide film.

### Step (c):

The aluminum plate from which the oxide film was removed was anodized in a 0.1 mol/L phosphoric acid aqueous solution for 90 seconds under the conditions of DC 195 V and a temperature of 0°C.

### Step (d):

The aluminum plate on which the oxide film was formed was immersed in 10% by mass phosphoric acid at 30°C for 35 minutes to expand a pore diameter.

### Step (e):

The aluminum plate which was subjected to the pore diameter expanding treatment was anodized in a 0.1 mol/L phosphoric acid aqueous solution for 90 seconds under the conditions of DC 195 V and a temperature of 0°C.

### Step (f):

The step (d) and the step (e) are repeated a total of four times to obtain an aluminum plate (test product) on which an oxide film (alumite) having a substantially conical recess portion with an average distance of 500 nm and a projection portion surrounded by the recess portion was formed. Table 1 shows an average distance, an average height, and an aspect ratio of the projection portions.

### [Comparative Example 1]

An aluminum plate on which an oxide film having substantially conical recess portions having an average distance of 100 nm was formed was prepared as a mold.

20 parts by mass of dipentaerythritol hexaacrylate, 70 parts by mass of bifunctional or higher hydrophilic (meth)acrylate (manufactured by TOAGOSEI CO., LTD., Aronix M-260, having an average repeating unit of polyethylene glycol chain of 13), 10 parts by mass of hydroxyethyl acrylate, and 1.5 parts by mass of 1-hydroxycyclohexylphenyl ketone (manufactured by BASF Japan, Irgacure (registered trademark) 184) were mixed to obtain an acrylic curable resin composition.

The acrylic curable resin composition was applied to a surface of the mold on a recess portion side, and a polyethylene terephthalate (hereinafter referred to as "PET") film having a thickness of 80 µm was put thereon.

Using an ultraviolet irradiator, irradiation with ultraviolet rays was performed through a PET film with an integrated light amount of 1,000 mJ/cm², and the acrylic curable resin composition was cured. Thereafter, the mold is separated to obtain a PET film (test product) in which a cured resin layer having a plurality of projection portions was formed on a surface. Table 1 shows an average distance, an average height, and an aspect ratio of the projection portions.

### [Comparative Example 2]

Except that an aluminum plate on which an oxide film having substantially conical recess portions having an average distance of 200 nm was formed was used as the mold, a PET film (test product) in which a cured resin layer having a plurality of projection portions was formed on a surface was obtained in the same manner as in Comparative Example 1. Table 1 shows an average distance, an average height, and an aspect ratio of the projection portions.

### [Comparative Example 3]

Except that an aluminum plate on which an oxide film having substantially conical recess portions having an average distance of 500 nm was formed was used as the mold, a PET film (test product) in which a cured resin layer having a plurality of projection portions was formed on a surface was obtained in the same manner as in Comparative Example 1. Table 1 shows an average distance, an average height, and an aspect ratio of the projection portions.

### [Antimicrobial test]

An antimicrobial test was performed for test products of Examples 1 to 4 and Comparative Examples 1 to 4 in accordance with JIS Z 2801: 2010 (corresponding international standard ISO 22196: 2007).

### 1. Test bacteria

Staphylococcus aureus: Staphylococcus aureus NBRC 12732
E. coli: Escherichia coli NBRC 3972

### 2. Preparation of test piece

A test product (50 mm × 60 mm) was immersed in 75% ethanol for 20 minutes and then sufficiently dried to obtain a test piece. Three test pieces were prepared for each example.

As an unprocessed test piece, the followings were prepared.

Examples 1 to 4 and Comparative Example 1: An aluminum plate (50 mm × 50 mm) was immersed in 75% ethanol for 20 minutes and then sufficiently dried to obtain an unprocessed test piece as a control product. Six unprocessed test pieces were prepared for each example.

Comparative Examples 1 to 3: A commercially available acrylic film was used as a target product. A control product (50 mm × 50 mm) was immersed in 75% ethanol for 20 minutes and then sufficiently dried to obtain an unprocessed test piece. Six unprocessed test pieces were prepared for each example.

### 3. Preparation of test bacterial solution

The test bacteria were transplanted to an ordinary agar medium and cultured at 35°C for 24 hours, and then one platinum loop was transplanted to the ordinary agar medium again and cultured at 35°C for 20 hours. The bacterial cells were uniformly dispersed in a 1/500 concentration ordinary bouillon medium to obtain a test bacterial solution.

### 4. Test operation

0.4 mL of the test bacterial solution was dropped on a processed surface of the test piece, and a polyethylene terephthalate plate (40 mm × 40 mm) treated in the same manner as the test piece was put on the test piece and pressed so that the test bacterial solution was spread over the entire surface. In addition, the same operation was performed on the unprocessed test pieces, and three test pieces and three unprocessed test pieces were allowed to stand at a temperature of 35°C and a relative humidity of 90% or higher for 24 hours. The remaining three unprocessed test pieces were used for measuring bacteria count immediately after inoculation with the test bacterial solution.

### 5. Bacteria count measurement

Each of the test pieces and the unprocessed test pieces after standing for 24 hours was placed in a sterile stomacher bag, 10 mL of SCDLP bouillon medium was added thereto, and the bacterial solution was thoroughly washed out to prepare a specimen. 1 mL of the specimen was cultured using a standard agar medium at 35°C for 48 hours, and then viable bacteria count was measured. The same operation was performed on the unprocessed test piece immediately after inoculation.

### 6. Antimicrobial activity value

An antimicrobial activity value was determined using the following formula. Results are shown in Table 1. A case where the antimicrobial activity value is 2.0 or more in both Staphylococcus aureus and Escherichia coli is determined to have an antimicrobial effect.

R=(Uₜ-U₀)-(Aₜ-U₀)

Here, Uo is an average value of logarithmic values of viable bacteria counts of the unprocessed test piece immediately after inoculation, Ut is an average value of logarithmic values of viable bacteria counts of the unprocessed test piece after 24 hours, and At is an average value of logarithmic values of viable bacteria counts of the test piece after 24 hours. The viable bacteria count was a value converted per 1 cm² of the test piece.

**[Table 1]**

| | Surface material | Recess portion | Projection portion | | | Antimicrobial activity value | |
|---|---|---|---|---|---|---|---|
| | | Average distance (nm) | Average distance (nm) | Average height (nm) | Aspect ratio | Staphylococcus aureus | Escherichia coli |
| Example 1 | Alumite | 100 | 58 | 180 | 3.1 | 3.4 | 3.7 |
| Example 2 | Alumite | 63 | 36 | 200 | 5.6 | 5.1 | 7.2 |
| Example 3 | Alumite | 200 | 115 | 600 | 5.2 | 5.4 | 4.4 |
| Example 4 | Alumite | 500 | 289 | 1800 | 6.2 | 5.1 | 3.9 |
| Comparative Example 1 | Acrylic cured product | - | 100 | 180 | 1.8 | 1.0 | 0.2 |
| Comparative Example 2 | Acrylic cured product | - | 200 | 200 | 1 | 0.1 | 0.2 |
| Comparative Example 3 | Acrylic cured product | - | 500 | 1400 | 2.8 | 0.2 | 0 |

### [Example 5]

An aluminum plate having a purity of 99.99% was polished by padding and electropolished in a perchloric acid/ethanol mixed solution (1/4 volume ratio) to be mirror-finished.

The electropolished aluminum plate was anodized in a 0.3 M sulfuric acid for 1 minute under the conditions of DC 25 V and a temperature of 17°C to obtain an aluminum plate with an anodic oxide coating.

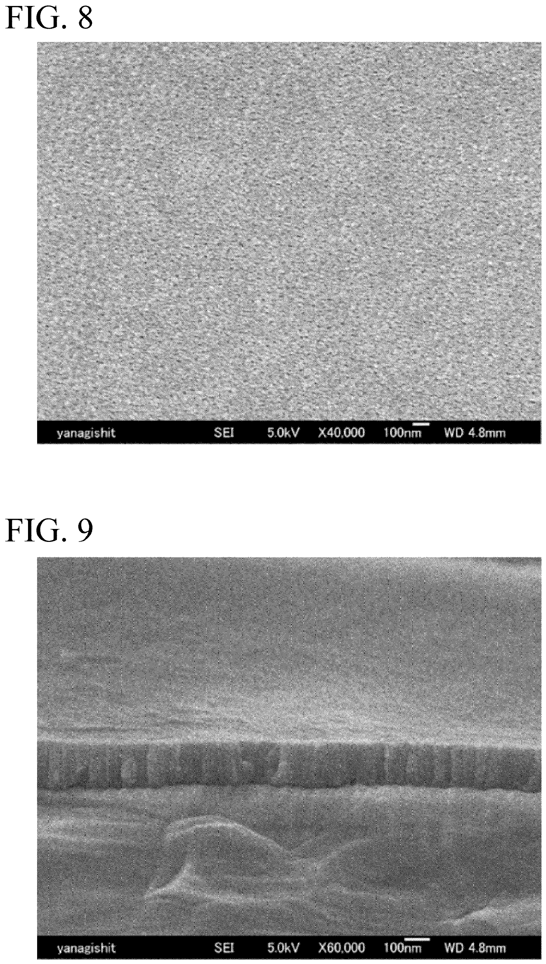

Fig. 8 shows a scanning electron microscope image of the surface of the aluminum plate with an anodic oxide coating, and Fig. 9 shows a scanning electron microscope image of a section of the aluminum plate with an anodic oxide coating.

The obtained aluminum plate with an anodic oxide coating was attached to a polyethylene plate to produce an antimicrobial layered body (test product).

### [Example 6]

An aluminum plate having a purity of 99.99% was polished by padding and electropolished in a perchloric acid/ethanol mixed solution (1/4 volume ratio) to be mirror-finished.

The electropolished aluminum plate was anodized in a 0.05 M oxalic acid aqueous solution for 100 seconds under the conditions of DC 80 V and a temperature of 17°C to obtain an aluminum plate with an anodic oxide coating.

The obtained aluminum plate with an anodic oxide coating was attached to a polyethylene plate to produce an antimicrobial layered body (test product).

### [Example 7]

An aluminum plate having a purity of 99.99% was polished by padding and electropolished in a perchloric acid/ethanol mixed solution (1/4 volume ratio) to be mirror-finished.

The electropolished aluminum plate was anodized in a 0.1 M phosphoric acid aqueous solution for 8 minutes under the conditions of DC 195 V and a temperature of 0°C to obtain an aluminum plate with an anodic oxide coating.

The obtained aluminum plate with an anodic oxide coating was attached to a polyethylene plate to produce an antimicrobial layered body (test product).

### [Comparative Example 4]

An aluminum plate (50 mm × 50 mm) obtained in a manner that an aluminum plate having a purity of 99.99% was polished by padding and electropolished in a perchloric acid/ethanol mixed solution (1/4 volume ratio) to be mirror-finished was used as a test product.

### [Comparative Example 5]

An acrylic film (50 mm × 50 mm) was used as a test product.

### [Antimicrobial test]

An antimicrobial test was performed for test products of Examples 5 to 7 and Comparative Examples 4 and 5 in accordance with JIS Z 2801: 2010 (corresponding international standard ISO 22196: 2007).

### 1. Test bacteria

Staphylococcus aureus: Staphylococcus aureus NBRC 12732
E. coli: Escherichia coli NBRC 3972

### 2. Preparation of test piece

A test product (50 mm × 60 mm) was immersed in 75% ethanol for 20 minutes and then sufficiently dried to obtain a test piece. Two test pieces were prepared for each example.

### 3. Preparation of test bacterial solution

The test bacteria were transplanted to an ordinary agar medium and cultured at 35°C for 24 hours, and then one platinum loop was transplanted to the ordinary agar medium again and cultured at 35°C for 20 hours. The bacterial cells were uniformly dispersed in an ordinary bouillon medium having 1/500 concentration to obtain a test bacterial solution.

### 4. Test operation

In Examples 5 to 7, 0.4 mL of the test bacterial solution was dropped on a processed surface of the test piece, and a polyethylene terephthalate plate (40 mm × 40 mm) treated in the same manner as the test piece was put on the test piece and pressed so that the test bacterial solution was spread over the entire surface.

In Comparative Examples 4 and 5, the same operation was performed on one side of the test piece.

Three test pieces were allowed to stand at a temperature of 35°C and a relative humidity of 90% or higher for 24 hours.

### 5. Bacteria count measurement

Each of the test pieces after standing for 24 hours was placed in a sterile stomacher bag, 10 mL of SCDLP bouillon medium was added thereto, and the bacterial solution was thoroughly washed out to prepare a specimen. 1 mL of the specimen was cultured using a standard agar medium at 35°C for 48 hours, and then viable bacteria count was measured.

Measurement results were shown in Table 1. The viable bacteria counts of Examples 5 to 7 and Comparative Examples 4 and 5 are average values of the results obtained from the two test pieces.

### 6. Measurement of initial bacteria count

An aluminum plate (50 mm × 50 mm) obtained in a manner that an aluminum plate having a purity of 99.99% was polished by padding and electropolished in a perchloric acid/ethanol mixed solution (1/4 volume ratio) to be mirror-finished was immersed in a 75% ethanol for 20 minutes, and then sufficiently dried to obtain an unprocessed test piece. Three unprocessed test pieces were prepared.

0.4 mL of the test bacterial solution was dropped on one surface of the unprocessed test piece, and a polyethylene terephthalate plate (40 mm × 40 mm) treated in the same manner as the test piece was put on the test piece and pressed so that the test bacterial solution was spread over the entire surface. Each of the unprocessed test piece was placed in a sterile stomacher bag, 10 mL of SCDLP bouillon medium was added thereto, and the bacterial solution was thoroughly washed out to prepare a specimen. 1 mL of the specimen was cultured using a standard agar medium at 35°C for 48 hours, and then viable bacteria count (initial bacteria count) was measured. Measurement results were shown in Table 1. The viable bacteria count is an average value of results obtained from three test pieces.

### 7. Evaluation of antimicrobial property

Those whose viable bacteria count was 1/100 or less (1% or less) of the initial bacteria count were evaluated as having an antimicrobial property. Evaluation was shown in Table 1.
Antimicrobial... A
Antimicrobial against some bacteria ... B
Non-antimicrobial... C

### [Explanation of results]

In Examples 5 and 6, the growth of Staphylococcus aureus and Escherichia coli was strongly suppressed. Example 5 in which the sulfuric acid was used as the electrolytic solution showed a particularly excellent antimicrobial property. Example 7 in which the phosphoric acid was used as the electrolytic solution was able to suppress the growth of Staphylococcus aureus, but had a weak effect on Escherichia coli.

An effect of the test product which showed a particularly strong effect and used the sulfuric acid as the electrolytic solution, was further verified under stricter conditions than those of JIS Z 2801: 2010.

### [Example 8]

An aluminum plate having a purity of 99.99% was polished by padding and electropolished in a perchloric acid/ethanol mixed solution (1/4 volume ratio) to be mirror-finished.

The electropolished aluminum plate was anodized in a 0.3 M sulfuric acid for 1 minute under the conditions of DC 25 V and a temperature of 17°C to obtain an aluminum plate with an anodic oxide coating.

The obtained aluminum plate with an anodic oxide coating was attached to a polyethylene plate to produce an antimicrobial layered body (test product).

### [Example 9]

An aluminum plate having a purity of 99.99% was polished by padding and electropolished in a perchloric acid/ethanol mixed solution (1/4 volume ratio) to be mirror-finished.

The electropolished aluminum plate was anodized in a 0.3 M sulfuric acid for 1 minute under the conditions of DC 25 V and a temperature of 17°C. Furthermore, a heat treatment (300°C, 10 minutes) was performed to obtain an aluminum plate with an anodic oxide coating.

The obtained aluminum plate with an anodic oxide coating was attached to a polyethylene plate to produce an antimicrobial layered body (test product).

### [Example 10]

An aluminum plate having a purity of 99.99% was polished by padding and electropolished in a perchloric acid/ethanol mixed solution (1/4 volume ratio) to be mirror-finished.

The electropolished aluminum plate was anodized in a 6 M sulfuric acid for 1 minute under the conditions of DC 25 V and a temperature of 17°C.

The obtained aluminum plate with an anodic oxide coating was attached to a polyethylene plate to produce an antimicrobial layered body (test product).

### [Example 11]

An aluminum plate having a purity of 99.99% was polished by padding and electropolished in a perchloric acid/ethanol mixed solution (1/4 volume ratio) to be mirror-finished.

The electropolished aluminum plate was anodized in a 8 M sulfuric acid for 1 minute under the conditions of DC 25 V and a temperature of 17°C to obtain an aluminum plate with an anodic oxide coating.

The obtained aluminum plate with an anodic oxide coating was attached to a polyethylene plate to produce an antimicrobial layered body (test product).

### [Example 12]

An aluminum plate having a purity of 99.99% was polished by padding and electropolished in a perchloric acid/ethanol mixed solution (1/4 volume ratio) to be mirror-finished.

The electropolished aluminum plate was anodized in a 12 M sulfuric acid for 1 minute under the conditions of DC 25 V and a temperature of 17°C to obtain an aluminum plate with an anodic oxide coating.

The obtained aluminum plate with an anodic oxide coating was attached to a polyethylene plate to produce an antimicrobial layered body (test product).

### [Example 13]

An aluminum layer having a thickness of 50 nm and a purity of 99.999% was deposited on a surface of a polypropylene film base material having a thickness of 0.2 mm (PP craft film PF-11 manufactured by Acrysunday Co., Ltd.) by a sputtering method to obtain an aluminum layered polypropylene film.

The obtained aluminum layered polypropylene film was anodized under the conditions of DC 25 V and a temperature 17°C while being slowly immersed in an electrolytic solution at a speed of 2 mm/min using a dip coater to produce an antimicrobial layered body (test product) in which the deposited aluminum layer was almost completely anodized. 12M sulfuric acid was used as the electrolytic solution. In the obtained layered body, the aluminum layer was completely anodized and was translucent.

### [Comparative Example 6]

An aluminum plate (50 mm × 50 mm) obtained in a manner that an aluminum plate having a purity of 99.99% was polished by padding and electropolished in a perchloric acid/ethanol mixed solution (1/4 volume ratio) to be mirror-finished was used as a test product.

### [Antimicrobial test]

An antimicrobial test was performed for test products of Examples 8 to 13 and Comparative Example 6 by partially modifying contents of JIS Z 2801: 2010 (corresponding international standard ISO 22196: 2007). Specifically, at the time of adjusting the bacterial solution, a concentration of the ordinary bouillon medium in which bacterial cells were uniformly dispersed was set to 1/100 concentration, which was 5 times higher than that of the JIS standard. Other than the above, the same operation as in Examples 5 to 7 was performed, and the viable bacteria count was measured. Measurement results were shown in Table 2. Note that, the viable bacteria count is the average value of the results obtained from the three test pieces.

**[Table 2]**

| | Material | Polybasic acid | Viable bacteria count (CFU/mL) | | Antimicrobial property |
|---|---|---|---|---|---|
| | | | Staphylococcus aureus | Escherichia coli | |
| Example 5 | Aluminum | 0.3 M Sulfuric acid | 2.5 | 0 | A |
| Example 6 | Aluminum | 0.05 M Oxalic acid | 1.5 | 33 | A |
| Example 7 | Aluminum | 0.1 M Phosphoric acid | 565 | 910000 | B |
| Comparative Example 4 | Aluminum | - | 175000 | 1650000 | C |
| Comparative Example 5 | Acrylic | - | 155000 | 2550000 | C |
| Initial bacteria count | Aluminum | - | 220000 | 350000 | - |

**[Table 3]**

| | Material | Polybasic acid | Heat treatment | Viable bacteria count (CFU/mL) | | Antimicrobial property | Bacterial survival rate (%) | |
|---|---|---|---|---|---|---|---|---|
| | | | | Staphylococcus aureus | Escherichia coli | | Staphylococcus aureus | Escherichia coli |
| Example 8 | Aluminum | 0.3 M Sulfuric acid | None | 533 | 44700 | B | 0.85 | 82.32 |
| Example 9 | Aluminum | 0.3 M Sulfuric acid | Performed | 233 | 38300 | B | 0.37 | 70.53 |
| Example 10 | Aluminum | 6 M Sulfuric acid | None | 0 | 12 | A | 0.00 | 0.02 |
| Example 11 | Aluminum | 8 M Sulfuric acid | None | 167 | 153 | A | 0.27 | 0.28 |
| Example 12 | Aluminum | 12 M Sulfuric acid | None | 0 | 0 | A | 0.00 | 0.00 |
| Example 13 | A1 layered plate | 12 M Sulfuric acid | None | 0 | 0 | A | 0.00 | 0.00 |
| Comparative Example 6 | Aluminum | - | None | 62567 | 54300 | C | 100.00 | 100.00 |
| Initial bacteria count | Aluminum | - | None | 21000 | 20667 | - | - | - |

### [Explanation of antimicrobial test results]

In a test conducted in an environment in which germs were more likely to grow than in JIS Z 2801: 2010 (corresponding international standard ISO 22196: 2007), Examples 8 and 9 were able to suppress the growth of Staphylococcus aureus, but had a weak effect on Escherichia coli. On the other hand, Examples 10 to 13 were able to strongly suppress the growth of both Staphylococcus aureus and Escherichia coli.

From Examples 8 and 9, there was no difference in antimicrobial property depending on the presence or absence of a heat treatment.

### [XPS analysis]

The surface of the test piece used in Examples 8 to 12 was measured under the following conditions, and an abundance ratio of each atom on the surface of the metal oxide layer was determined.

X-ray photoelectron spectroscopy analyzer: Quantum-2000 manufactured by ULVAC-PHI
X-ray source: Monochromated-Al-Kα ray (output 16 kV, 34 W)
Extraction angle: 45°
Measurement area: 300 µm□

The results obtained are shown in Table 4. The numbers in the tables represent atm%.

### [Explanation of XPS analysis results]

S derived from an anion of the sulfuric acid electrolytic solution was detected on the surface of the test piece, which showed an effect even in the test conducted in an environment in which germs were more likely to grow than in JIS Z 2801: 2010 (corresponding international standard ISO 22196: 2007). Furthermore, it was clear that the amount of an element derived from an anion derived from an electrolytic solution on a surface can be increased using the concentration of the electrolytic solution used for anodizing. In addition, in Examples 8 and 9, there was no significant difference in a surface state depending on the presence or absence of a heat treatment. In addition, as for a peak position of C, a sharp peak is observed at 285 ev derived from C-C or C-H bonds, and no conspicuous peak was observed at 290 ± 1.3 ev derived from a carboxyl group. Therefore, it was determined that C was not derived from an anion but was derived from dirt or the like adhering to the surface.

**[Table 4]**

| Element | C | O | Al | S |
|---|---|---|---|---|
| Example 8 | 14.2 | 58.2 | 25.4 | 2.2 |
| Example 9 | 14.7 | 59.6 | 23.7 | 2 |
| Example 10 | 2.5 | 68.1 | 26.2 | 3.2 |
| Example 11 | 13.4 | 60.3 | 23.3 | 3.1 |
| Example 12 | 14.2 | 59.5 | 22.5 | 3.8 |

| | | | | |
|---|---|---|---|---|
| Unit: atm% | | | | |

### [Measurement of transmittance]

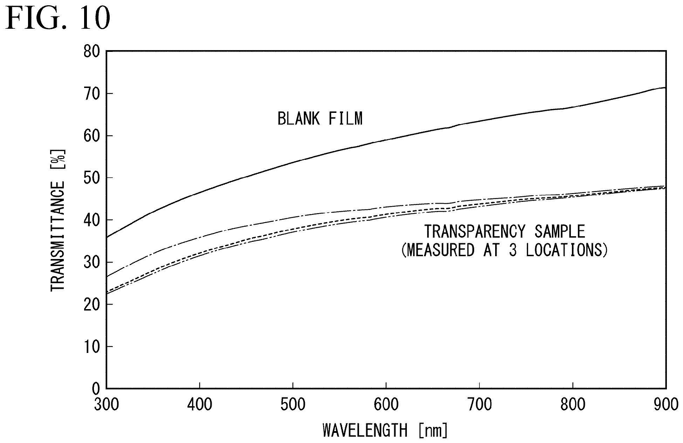

A total light transmittance of the antimicrobial layered body obtained in Example 13 and the polypropylene film base material (blank film) used for producing the same was measured. For the measurement, a haze meter (manufactured by Suga Test Instruments Co., Ltd.) in accordance with JIS K 7136: 2000 was used. Results are shown in Fig. 10. As was clear from the results, the antimicrobial layered body obtained in Example 13 transmitted light in all wavelength bands of visible light.

### [Antimicrobial test]

An antimicrobial test was performed for an acrylic plate, an aluminum plate, and a test product of Examples 13 by partially modifying contents of JIS Z 2801: 2010 (corresponding international standard ISO 22196: 2007). Specifically, at the time of adjusting the bacterial solution, the antimicrobial test was performed at a medium concentration by setting the concentration of the ordinary bouillon medium in which bacterial cells were uniformly dispersed to 1/50 concentration, which was 10 times higher, 1/20 concentration, which was 25 times higher, or 1/10 concentration, which was 50 times higher than that of the JIS standard. Measurement results were shown in Table 5. Note that, the viable bacteria count is the average value of the results obtained from the three test pieces.

**[Table 5]**

| | | Viable bacteria count (CFU/mL) | |
|---|---|---|---|
| | | Staphylococcus aureus | Escherichia coli |
| 50 times medium concentration | Acrylic plate | 1533333 | 11333333 |
| | Aluminum plate | 1300000 | 9833333 |
| | Example 13 | 3.0 | 1.7 |
| 25 times medium concentration | Acrylic plate | 590000 | 6000000 |
| | Aluminum plate | 320000 | 4700000 |
| | Example 13 | 0.0 | 2.7 |
| 10 times medium concentration | Acrylic plate | 326667 | 2966667 |
| | Aluminum plate | 320000 | 4700000 |
| | Example 13 | 1.7 | 0.0 |
| Initial bacteria count | | 30667 | 36000 |

### [Explanation of antimicrobial test results]

Even in a test conducted in an environment in which germs were much more likely to grow than in JIS Z 2801: 2010 (corresponding international standard ISO 22196: 2007), the antimicrobial layered body of Example 13 was able to strongly suppress the growth of both Staphylococcus aureus and Escherichia coli.

### [Industrial Applicability]

The antimicrobial material and the antimicrobial layered body of the present invention can exhibit an excellent antimicrobial effect without using a drug, and thus are useful as a medical member, a food packaging material, and the like.

### [Reference Signs List]

10 Antimicrobial material
12 Aluminum base material
14 Oxide film
16 Cell
18 Recess portion
20 Projection portion
22 Ridge portion
24 Pore
26 Pore generation point
30 Layered body
32 Layer made of antimicrobial material
34 Other base material
110 Aluminum plate with anodic oxide coating
112 Aluminum base material
114 Oxide film
124 Pore

## Claims

1. An antimicrobial layered body comprising:
a non-metal substrate; and
a metal oxide layer,
wherein the metal oxide layer is present on an outermost surface,
the metal oxide layer contains an anion, and
a total abundance ratio of at least one atom of a sulfur atom, a phosphorus atom, and a carbon atom which are derived from the anion is 1.0 atm% or more when analyzed by XPS.

2. The antimicrobial layered body according to Claim 1,
wherein the anion is selected from the group consisting of SO₄, PO₄³⁻, C₂O₄²⁻, C₃H₂O₄²⁻, C₄H₄O₅²⁻, and C₆H₅O₇³⁻.

3. The antimicrobial layered body according to Claim 1 or 2,
wherein, in the metal oxide layer present on the outermost surface, an abundance ratio of at least one atom of a sulfur atom, a phosphorus atom, and a carbon atom derived from the anion is 3.0 atm% or more.

4. The antimicrobial layered body according to any one of Claims 1 to 3, further comprising:
a metal layer.

5. The antimicrobial layered body according to any one of Claims 1 to 4,
wherein a metal contained in the metal oxide layer and a metal layer is a valve metal.

6. The antimicrobial layered body according to Claim 5,
wherein the valve metal is aluminum.

7. The antimicrobial layered body according to Claim 5 or 6,
wherein, in the metal oxide layer present on the outermost surface, a total abundance ratio of the valve metal is 10 atm% or more when analyzed by XPS, and a total abundance ratio of a non-valve metal and a halogen atom is 1.0 atm% or less when analyzed by XPS.

8. The antimicrobial layered body according to Claim 7,
wherein the non-valve metal is at least one selected from the group consisting of silver, copper, titanium, and germanium, and
the halogen atom is an iodine atom.

9. The antimicrobial layered body according to any one of Claims 1 to 8,
wherein, in the metal oxide layer present on the outermost surface, an atom derived from the anion is a sulfur atom and an abundance ratio of an oxygen atom is 45 atm% or more when analyzed by XPS.

10. The antimicrobial layered body according to any one of Claims 1 to 9,
wherein a total light transmittance of the metal oxide layer is 30% or more.

11. The antimicrobial layered body according to any one of Claims 1 to 10,
wherein a thickness of the metal oxide layer is 50 nm or larger and 10 µm or smaller.

12. The antimicrobial layered body according to any one of Claims 1 to 11,
wherein the metal oxide layer present on the outermost surface has a plurality of projection portions on the outermost surface thereof, and
an average distance between adjacent projection portions is 20 to 600 nm.

13. The antimicrobial layered body according to Claim 12,
wherein the projection portion is a needle-like protrusion.

14. An antimicrobial layered body production method which is for the antimicrobial layered body according to any one of Claims 1 to 13, the method comprising:
a step of anodizing a surface of a metal layer containing 95% by mass or more of a valve metal by using a polybasic acid having a concentration of 0.04 M or higher to form the metal oxide layer.

15. The antimicrobial layered body production method according to Claim 14,
wherein a concentration of the polybasic acid is 0.3 M or higher.

16. The antimicrobial layered body production method according to Claim 14 or 15,
wherein the valve metal is aluminum, and
the polybasic acid is sulfuric acid having a concentration of 3 M or higher.

17. The antimicrobial layered body production method according to Claim 16,
wherein a concentration of the sulfuric acid is 6 M or higher.

18. An antimicrobial material comprising:
an oxide film of a metal having a plurality of projection portions on a surface,
wherein an average distance between adjacent projection portions is 20 to 400 nm.

19. The antimicrobial material according to Claim 18,
wherein the projection portion is a needle-like protrusion.

20. A layered body comprising:
a layer made of the antimicrobial material according to Claim 18 or 19.

21. A medical member comprising:
the antimicrobial material according to Claim 18 or 19.

22. An antimicrobial material production method for producing the antimicrobial material according to Claim 18 or 19, the method comprising:
forming an oxide film having a plurality of projection portions on a surface by performing, once or more times, a combination of a step of anodizing a metal base material to form an oxide film having a pore and a step of expanding a diameter of the pore.

23. An antimicrobial method comprising:
providing the antimicrobial material according to Claim 18 or 19 at a place where growth of bacteria is desired to be suppressed.
